# EUROPÄISCHE PATENTANMELDUNG

(11) **EP 3 753 582 A1**
(43) Veröffentlichungstag der Anmeldung: **23.12.2020**
(21) Anmeldenummer: 20175614.5
(22) Anmeldetag: 20.05.2020
(51) Int. Cl.: A61L 2/10, C02F 1/32

(54) **VORRICHTUNG UND VERFAHREN ZUM DESINFIZIEREN EINES FLUIDS MITTELS UV-LICHT**

(30) Priorität: 20.06.2019 DE 102019116745
(71) Anmelder: Hytecon AG, 6006 Luzern (CH)
(72) Erfinder: KLINK, Maximilian, 6006 Luzern (DE)
(74) Vertreter: Bittner, Thomas L.

(57) **Zusammenfassung**

Die Erfindung betrifft eine Vorrichtung zum Desinfizieren eines Fluids mittels UV-Licht, mit: einem Behälter (1); einem Reaktorraum (3), welcher in dem Behälter (1) angeordnet und eingerichtet ist, ein zu desinfizierendes Fluid aufzunehmen; einem Einlass (4), über welchen das Fluid in dem Reaktorraum (3) eingebracht werden kann; einem Auslass (5), über welchen das Fluid den Reaktorraum (3) verlassen kann; und einer Bestrahlungseinrichtung (2), die eingerichtet ist, UV-Lichtstrahlen bereitzustellen und zum Desinfizieren des Fluids im Reaktorraum (3) in diesen einzustrahlen. Der Reaktorraum (3) mit dem Einlass (4) und dem Auslass (5) ist eingerichtet, das Fluid mittels einer turbulenten Strömung von Einlass (4) zum Auslass (5) zu transportieren und eine das Ausbilden der turbulenten Strömung unterstützende Fluidleiteinrichtung (9) vorgesehen ist, welche in einem Reaktorraumbereich benachbart zum Einlass (4) ein Fluidleitelement (10) aufweist, das eine nicht turbulente Strömung zum Auslass (5) hin minimierend und hierdurch die turbulente Strömung verstärkend ausgebildet ist. Weiterhin ist ein Verfahren zum Desinfizieren eines Fluids mittels UV-Licht geschaffen.

## Beschreibung

Die Erfindung betrifft eine Vorrichtung und ein Verfahren zum Desinfizieren eines Fluids mittels UV-Licht.

### Hintergrund

UV-Licht, also ultraviolettes Licht, kann genutzt werden, um ein Fluid zu desinfizieren, zum Beispiel Wasser, insbesondere Brauch- oder Trinkwasser.

Aus dem Dokument WO 2017 / 045 662 A1 sind eine Vorrichtung zum Desinfizieren eines Fluids mittels UV-Licht sowie deren Verwendung bekannt. Bei der bekannten Vorrichtung ist ein Behälter mit einem Reaktorraum vorgesehen, in welchem das zu desinfizierende Fluid über einen Einlass eingebracht wird und aus dem das dann mittels UV-Licht Bestrahlung desinfiziertes Fluid über einen Auslass abgegeben werden kann. Hierbei ist vorgesehen, dass das Fluid in dem Reaktorraum als rotierender Fluidstrudel ausgebildet wird. Im Bereich einer Seitenwand des Reaktorraums sind UV-Lichtquellen angeordnet, die UV-Lichtstrahlen zum Desinfizieren auf den rotierenden Fluidstrudel einstrahlen. In einer Ausführungsform ist der Auslass bei der bekannten Vorrichtung an einem Rohrabschnitt gebildet, der in den Reaktorraum hineinragt.

### Zusammenfassung

Aufgabe der Erfindung ist es, eine Vorrichtung und ein Verfahren zum Desinfizieren eines Fluids mittels UV-Licht anzugeben, bei denen die Effizienz beim Desinfizieren des Fluids mittels des UV-Lichts verbessert ist.

Zur Lösung sind eine Vorrichtung sowie ein Verfahren zum Desinfizieren eines Fluids mittels UV-Licht nach den unabhängigen Ansprüchen 1 und 12 vorgesehen. Ausgestaltungen sind Gegenstand von abhängigen Unteransprüchen.

Nach einem Aspekt ist eine Vorrichtung zum Desinfizieren eines Fluids mittels UV-Licht geschaffen, die Folgendes aufweist: einen Behälter; einen Reaktorraum, welcher in dem Behälter angeordnet und eingerichtet ist, ein zu desinfizierendes Fluid aufzunehmen; einen Einlass, über welchen das Fluid in dem Reaktorraum eingebracht werden kann; einen Auslass, über welchen das Fluid den Reaktorraum verlassen kann; und eine Bestrahlungseinrichtung, die eingerichtet ist, UV-Lichtstrahlen bereitzustellen und zum Desinfizieren des Fluids im Reaktorraum in diesen einzustrahlen. Der Reaktorraum ist mit dem Einlass und dem Auslass eingerichtet, das Fluid mittels einer turbulenten Strömung vom Einlass zum Auslass zu transportieren. Es ist eine das Ausbilden der turbulenten Strömung unterstützende Fluideinrichtung vorgesehen, welche in einem Reaktorraumbereich benachbart zum Einlass ein Fluidleitelement aufweist, das eine nichtturbulente Strömung zum Auslass hin minimierend und hierdurch die turbulente Strömung verstärkend ausgebildet ist.

Nach einem weiteren Aspekt ist ein Verfahren zum Desinfizieren eines Fluids mittels UV-Licht unter Verwendung der Vorrichtung vorgesehen. Das Fluid wird durch einen Einlass in einem Reaktorraum eines Behälters der Vorrichtung eingebracht und in dem Reaktorraum zum einem Auslass transportiert. Das Fluid wird im Reaktorraum mittels UV-Lichtstrahlen desinfiziert, die von einer Bestrahlungseinrichtung in den Reaktorraum eingestrahlt werden. Das Fluid wird in dem Reaktorraum mittels einer turbulenten Strömung vom Einlass zum Auslass transportiert. Hierbei unterstützt eine Fluidleiteinrichtung das Ausbilden der turbulenten Strömung, wobei hierbei mittels eines Fluidleitelements in einem Reaktorraumbereich benachbart zum Einlass eine nicht turbulente Strömung zum Auslass hin minimiert und hierdurch das Ausbilden der turbulenten Strömung verstärkt wird.

Mittels des Fluidleitelements kann im Reaktorraum die nicht turbulente Strömung zum Auslass hin im Wesentlichen vollständig verhindert sein. Bei der nichtturbulenten Strömung kann es sich zum Beispiel um eine laminare Strömung entlang des Rohrabschnitts handeln.

Die Fluidleiteinrichtung kann mehrere Fluidleitelemente aufweisen.

Aufgrund der bei bekannten Vorrichtungen auftretenden nicht turbulenten Strömung zum Auslass hin, wird die Aufenthaltsdauer des Fluids in dem Reaktorraum gemindert, was die Effizienz des Desinfizierens mittels des UV-Lichts einschränkt. Dieses wird mit Hilfe des vorgesehenen Fluidleitelements verbessert, welches minimiert oder verhindert, dass ein Teil des Fluids, ohne von der turbulenten Strömung in der Reaktorkammer erfasst zu werden, zum Auslass hin strömt.

Die Bestrahlungseinrichtung kann zum Beispiel mit lichtimitierenden Dioden (LED) gebildet sein, die als UV-LEDs UV-Lichtstrahlen bereitstellen und in den Reaktorraum einstrahlen.

Der Reaktorraum kann zum Beispiel als ein zylindrischer Innenraum in dem Behälter ausgebildet sein.

Der Reaktorraum kann mit einem die UV-Lichtstrahlen diffus streuenden Material zumindest abschnittsweise beschichtet oder ausgekleidet sein, zum Beispiel Teflon.

Ist der Einlass im Bereich eines Wandabschnitts des Reaktorraums angeordnet, so kann das Fluidleitelement benachbart hierzu angeordnet sein, beispielsweise in einem Boden- oder einem Endbereich, wenn der Einlass zum Beispiel im Bereich des Bodens oder eines Endes des Reaktorraums angeordnet ist.

Die turbulente Strömung in dem Reaktorraum kann Wirbel umfassen, insbesondere Strudel. Aufgrund der turbulenten Strömung kann das Fluid auf seinem Weg vom Einlass zum Auslass mehrfach in dem Reaktorraum umlaufen, was die Zeitdauer für die UV-Lichtbestrahlung weiter erhöht.

Bei der Vorrichtung kann das Fluidleitelement in Bezug auf den Auslass von diesem beabstandet in einem distalen Bereich des Rohrabschnitts angeordnet sein. Erstreckt sich der Rohrabschnitt beispielsweise vom Boden oder von einem Deckel in den Reaktorraum hinein, kann das Fluidleitelement proximal zum Boden oder zum Deckel angeordnet sein.

Bei der Vorrichtung kann das Fluidleitelement in den Reaktorraum hineinragende und dort freistehend angeordnete Blatt- oder Flügelelemente aufweisen, die sich wahlweise, von einem mittleren Bereich des Reaktorraums ausgehend, bis in einen Bereich benachbart zur inneren Oberfläche des Reaktorraums erstrecken können. Es können mehrere Blatt- oder Flügelelemente in im Wesentlichen äquidistanten Abständen vorgesehen sein. Die Blatt- oder Flügelelemente können dreidimensional geformt sein, beispielsweise Flüge- oder Rotorblättern ähnlich.

Das Fluidleitelement kann in dem Reaktorraum verlagerbar angeordnet sein. Hierbei kann zum Beispiel vorgesehen sein, dass das Fluidleitelement frei oder selbsttätig verlagerbar in dem Reaktorraum angeordnet ist, zum Beispiel als freidrehbares Flügelrad. Alternativ kann vorgesehen sein, dass das Fluidleitelement in mehrere wählbare Stellungen verlagert werden kann, in denen es jeweils feststehend ist, zum Beispiel mittels einer Verrastung. In einer Ausführungsform kann vorgesehen sein, dass das Fluidleitelement höhenverlagerbar an einem Rohrabschnitt angeordnet ist. Alternativ ist das Fluidleitelement feststehend und nicht verlagerbar Reaktorraum angeordnet.

Das Fluidleitelement kann in dem Reaktorraum dem Einlass gegenüberliegend angeordnet sein. Auf diese Weise kann beispielsweise vorgesehen sein, dass das durch den Einlass im Reaktorraum eingebrachte Fluid auf das Fluidleitelement eingestrahlt wird oder auf dieses (direkt) zufließt.

Das Fluidleitelement kann in dem Reaktorraum im eine Drehachse drehbar angeordnet ist. Das über den Einlass in dem Reaktorraum eingebrachte Fluid kann zum Beispiel im Wesentlich axial zur Drehachse oder quer hierzu einströmen.

Der Auslass kann an einem Rohrabschnitt angeordnet sein. Hierbei kann bei verlagerbar gelagertem Fluidleitelement eine Drehbarkeit um eine Drehachse vorgesehen sein, die in Längsrichtung des Rohrabschnitts verläuft.

Der Rohrabschnitt kann von einem Wandabschnitt des Reaktorraums ausgehend in den Reaktorraum hineinragend gebildet sein, und der Auslass kann an einem in den Reaktorraum hineinragenden Ende des Rohrabschnitts angeordnet sein. Der Rohrabschnitt kann bei dieser Ausführungsform im Wesentlichen zentral oder mittig in dem Reaktorraum angeordnet sein, derart, dass das Fluid in dem Reaktorraum um den Rohrabschnitt herum transportiert werden kann. Beispielsweise kann sich der Rohrabschnitt von einem Boden oder einem Deckelabschnitt des Behälters in den Reaktorraum hinein erstrecken.

Das Fluidleitelement kann um den Rohrabschnitt herum ausgebildet sein. Das Fluidleitelement kann um den Rohrabschnitt herum unterbrochen oder umlaufend durchgehend ausgebildet sein.

Weitere der Fluidleitelemente können am und / oder benachbart zum Rohrabschnitt angeordnet sein, um so die nicht turbulente Strömung entlang des Rohrabschnitts zu minimieren oder ganz zu verhindern.

Der Auslass kann an dem Rohrabschnitt im Bereich einer Stirnfläche angeordnet sein.

Das Fluidleitelement kann eine nicht turbulente Strömung entlang einer äußeren Oberfläche des Rohrabschnitts zum Auslass hin minimierend und hierdurch die turbulente Strömung verstärkend ausgebildet sein.

In Verbindung mit dem Verfahren zum Desinfizieren des Fluids mittels UV-Licht können die vorangehend im Zusammenhang mit der Vorrichtung erläuterten Ausgestaltungen entsprechend vorgesehen sein.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung eines Behälters für eine Vorrichtung zum Desinfizieren eines Fluids mittels UV-Licht;
- Fig. 2: eine schematische Schnittdarstellung des Behälters aus Fig. 1 entlang einer Schnittlinie AA in Fig. 1;
- Fig. 3: eine schematische Schnittdarstellung des Behälters aus Fig. 1 entlang einer Schnittlinie BB in Fig. 1;
- Fig. 4: eine schematische Darstellung eines weiteren Behälters für eine Vorrichtung zum Desinfizieren eines Fluids mittels UV-Licht;
- Fig. 5: eine schematische Schnittdarstellung des Behälters aus Fig. 4 entlang einer Schnittlinie AA in Fig. 4;
- Fig. 6: eine schematische Schnittdarstellung des Behälters aus Fig. 4 entlang einer Schnittlinie BB in Fig. 4;
- Fig. 7: eine schematische Darstellung eines anderen Behälters für eine Vorrichtung zum Desinfizieren eines Fluids mittels UV-Licht;
- Fig. 8: eine schematische Schnittdarstellung des Behälters aus Fig. 7 entlang einer Schnittlinie AA in Fig. 4 und
- Fig. 9: eine schematische Schnittdarstellung des Behälters aus Fig. 7 entlang einer Schnittlinie BB in Fig. 4.

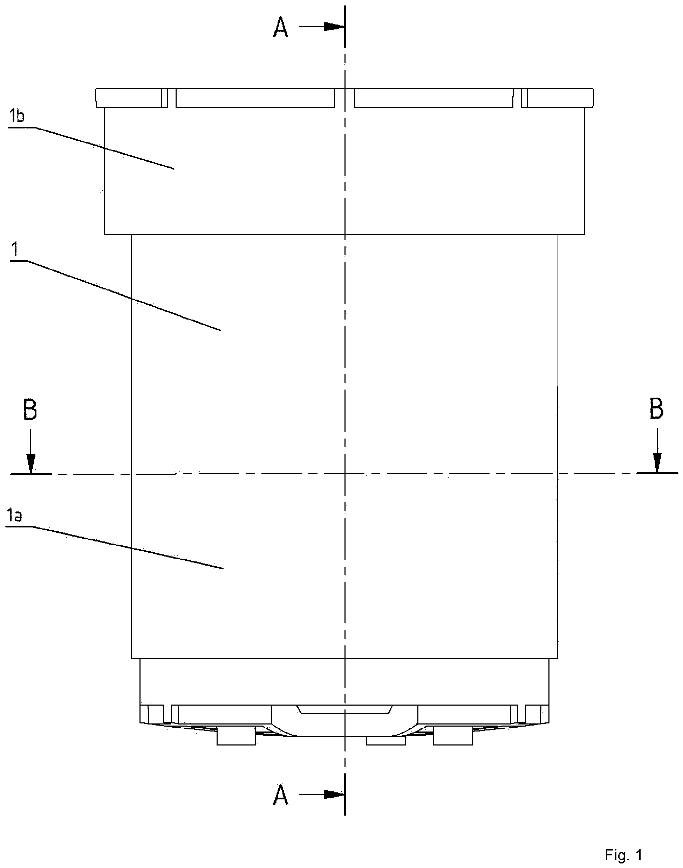

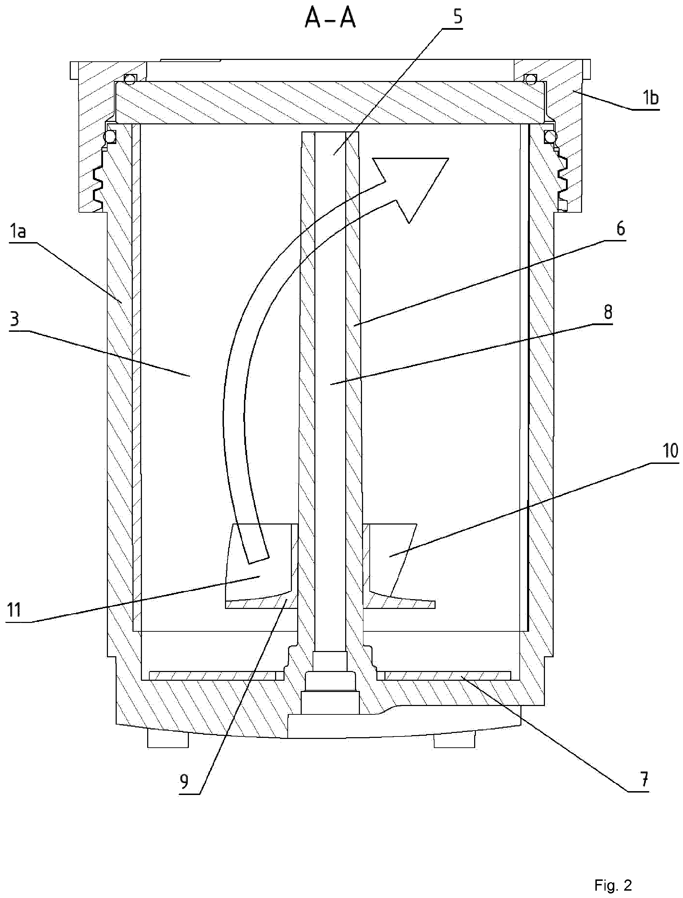

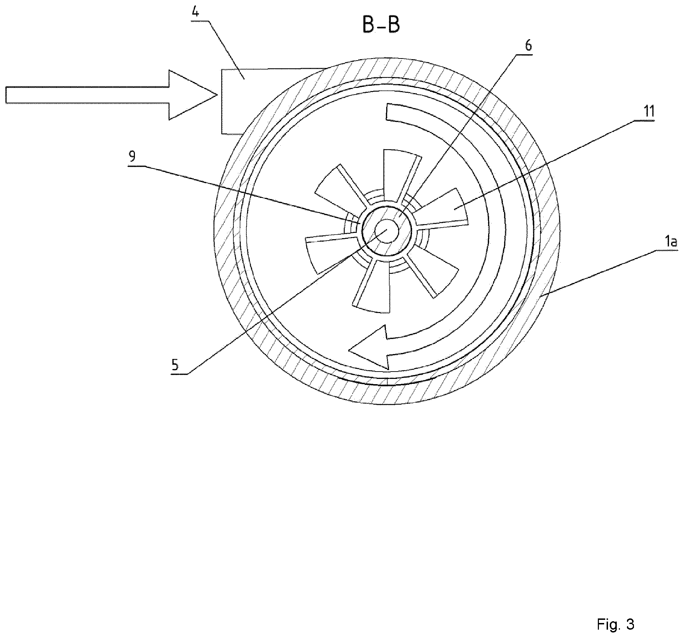

Fig. 1 zeigt eine schematische Darstellung eines Behälters 1 für eine Vorrichtung zum Desinfizieren eines Fluids mittels UV-Licht. Die Fig. 2 und 3 zeigen Schnittdarstellungen entlang der Schnittlinien AA und BB in Fig. 1.

Eine Bestrahlungsvorrichtung 2, die UV-Lichtstrahlen zum Desinfizieren eines Fluids in einem Reaktorraum 3 des Behälters 1 bereitstellt und beispielsweise mit UV-LEDs gebildet ist, ist mittels gestrichelter Linien schematisch angedeutet.

Das zu desinfizierende Fluid, insbesondere Wasser, zum Beispiel Trinkwasser, gelangt über einen Einlass 4 in den Reaktorraum 3. Das Fluid strömt hierbei derart ein, dass in dem Reaktorraum 3 eine turbulente Strömung entsteht, die insbesondere bewirkt, dass das Fluid in dem Reaktorraum 3 mehrfach umläuft, sodass die Verweildauer für das Fluid in dem Reaktorraum 3 optimiert ist, um die UV-Lichtbestrahlung zum Desinfizieren zu nutzen.

Nachdem das Fluid in dem Reaktorraum 3 nach oben gelangt ist, kann es den Reaktorraum 3 durch einen Auslass 5 verlassen, welcher stirnseitig an einem Rohrabschnitt 6 gebildet ist, der seinerseits in den Reaktorraum 3 vom Boden 7 ausgehend hineinragt und in dem ein Abflusskanal 8 bereitgestellt ist, durch den das desinfizierte Fluid dann einer weiteren Verwendung zugeführt werden kann, zum Beispiel einer Wasserabgabestelle.

Der Behälter 1 weist einen Topfbehälter 1a sowie einen Deckel 1b auf, welcher bei der gezeigten Ausführungsform aufgeschraubt ist.

Um das Ausbilden der turbulenten Strömung des Fluids in dem Reaktorraum 3 zu unterstützen, ist eine Fluidleiteinrichtung 9 mit einem Fluidleitelement 10 vorgesehen, welches an dem Rohrabschnitt 6 umlaufend gebildet ist und bei der gezeigten Ausführungsform Blatt- oder Flügelelemente 11 aufweist, die um den Rohrabschnitt 6 herum in gleichen Abständen angeordnet sind und sich von dem Rohrabschnitt 6 ausgehend in den Reaktorraum 3 erstreckend angeordnet sind. Mit Hilfe des Fluidleitelements 10 wird eine nichtturbulente Strömung des im Reaktorraum 3 eingebrachten Fluids entlang der Oberfläche des Rohrabschnitts 6 zum Auslass hin gemindert oder im Wesentlichen ganz unterbunden. Vielmehr unterstützt das Fluidleitelement 10 das Ausbilden der turbulenten Strömung, sodass das eingebrachte Fluid in möglichst großem Umfang hiervon erfasst wird. Während das Fluid beim Entlangfließen an der Oberfläche des Rohrabschnitts 6 kurzfristig zum Auslass 5 hin gelangt, ist dies bei der gezeigten Vorrichtung mit Hilfe des Fluidleitelements 10 verhindert.

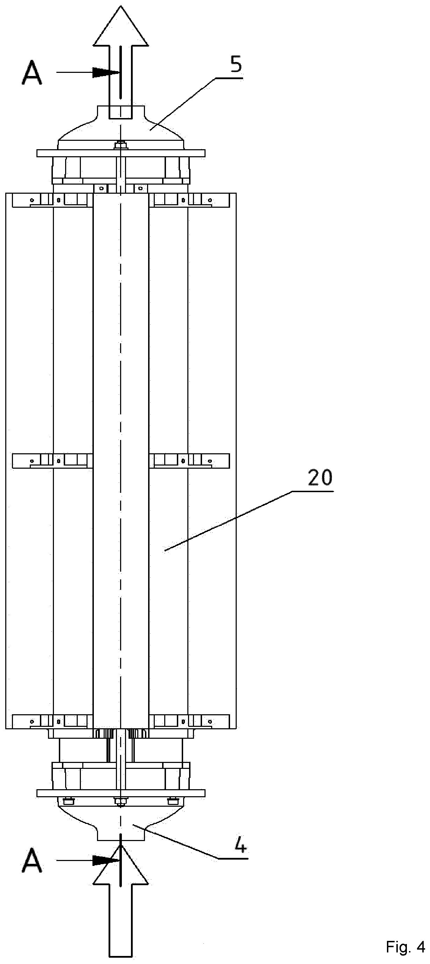

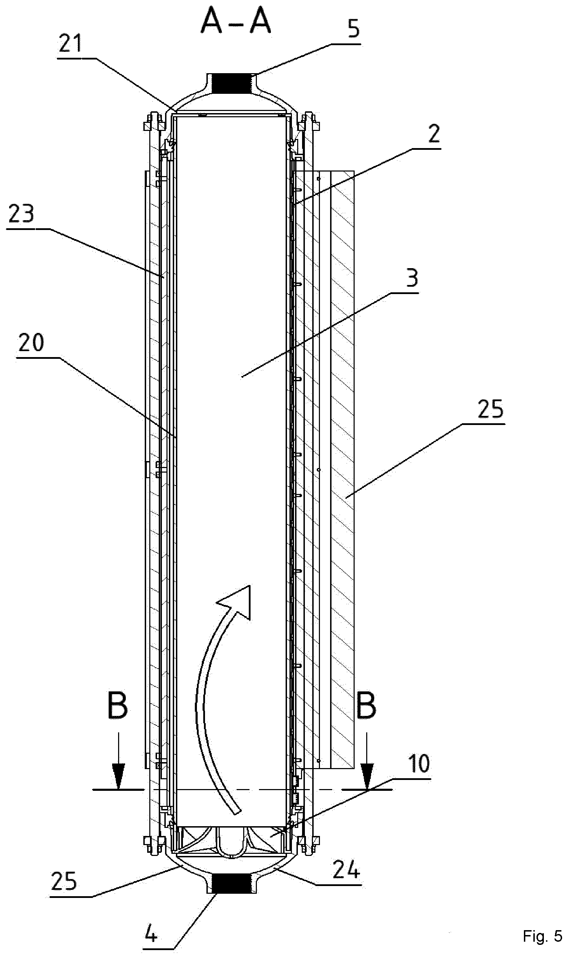

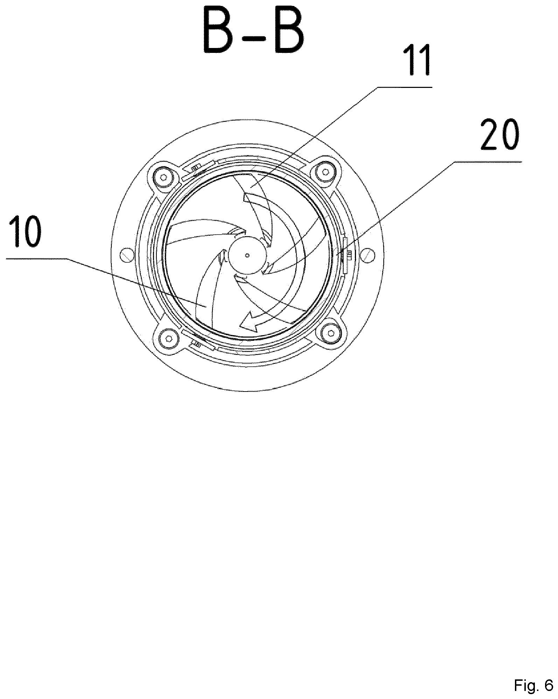

Fig. 4 zeigt eine schematische Darstellung eines weiteren Behälters 20 für eine Vorrichtung zum Desinfizieren eines Fluids mittels UV-Licht. Die Fig. 5 und 6 zeigen Schnittdarstellungen entlang der Schnittlinien AA und BB in Fig. 4. Für gleiche Merkmale werden in den Fig. 4 bis 6 dieselben Bezugszeichen wie in den Fig. 1 bis 3 verwendet.

Bei der Ausführungsform in den Fig. 4 bis 6 sind der Einlass 4 und der Auslass 5 im Bereich gegenüberliegender Enden 21, 22 des weiteren Behälters 20 angeordnet, der mit einem Rohr 23 gebildet ist, welches zum Beispiel aus Quarzglasrohr ist. Das Fluidleitelement 10, welches als Blatt- oder Flügelrad ausgeführt ist, liegt dem Einlass 4 gegenüber im Bereich eines Bodens 24. Das über den Einlass 4 einströmende Fluid trifft im Wesentlich quer zur Ebene, in welcher die Blattelemente 11 angeordnet sind, auf das Fluidleitelement 10.

Die das UV-Licht zum Desinfizieren bereitstellende Bestrahlungseinrichtung 2 ist um den Reaktorraum 3 gebildet und strahlt von außen in dem Reaktorraum 3 ein, wobei UV-LEDs zu Einsatz kommen können. Ein Kühlkörper 25, zum Beispiel ein Aluminiumkühler, kühlt die Bestrahlungseinrichtung 2 im Betrieb.

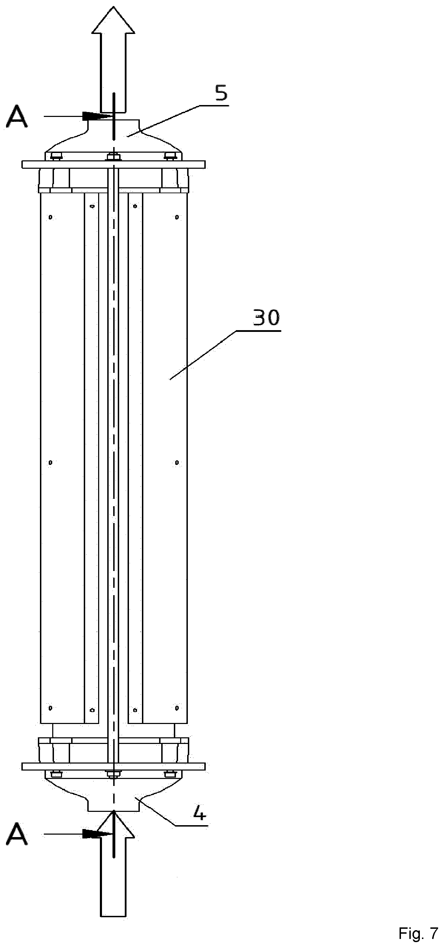

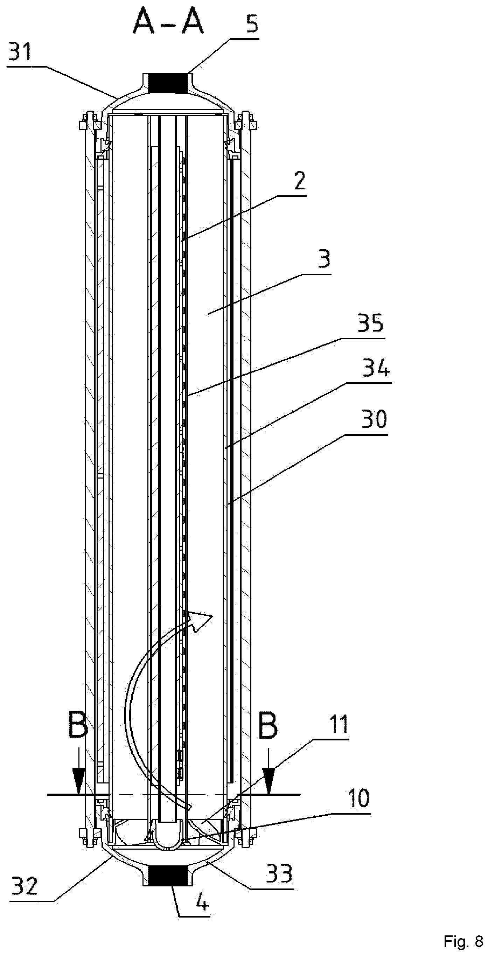

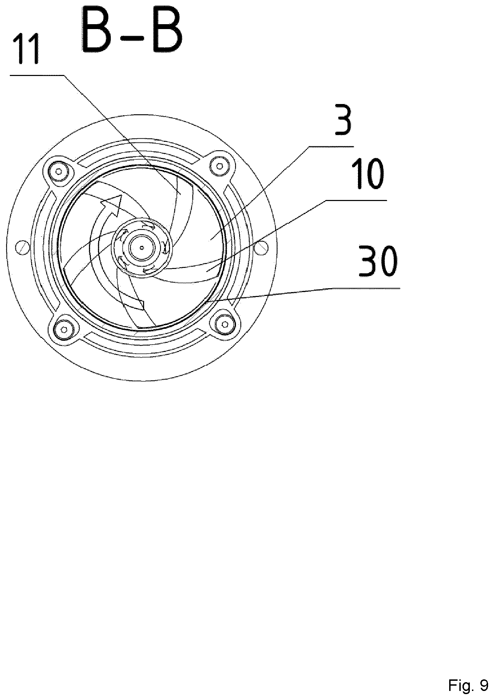

Fig. 7 zeigt eine schematische Darstellung eines anderen Behälters 30 für eine Vorrichtung zum Desinfizieren eines Fluids mittels UV-Licht. Die Fig. 8 und 9 zeigen Schnittdarstellungen entlang der Schnittlinien AA und BB in Fig. 7. Für gleiche Merkmale werden in den Fig. 7 bis 9 dieselben Bezugszeichen wie in den Fig. 1 bis 3 verwendet.

Bei der Ausführungsform in den Fig. 7 bis 9 sind der Einlass 4 und der Auslass 5 im Bereich gegenüberliegender Enden 31, 32 des anderen Behälters 30 angeordnet. Das Fluidleitelement 10, welches als Blatt- oder Flügelrad ausgeführt ist, liegt dem Einlass 4 gegenüber im Bereich eines Bodens 33. Das über den Einlass 4 einströmende Fluid trifft im Wesentlich quer zur Ebene, in welcher die Blattelemente 11 angeordnet sind, auf das Fluidleitelement 10.

Die das UV-Licht zum Desinfizieren bereitstellende Bestrahlungseinrichtung 2 ist in dem Reaktorraum 3 selbst angeordnet und wird von dem Fluid umspült, wenn dieses zum Auslass hin transportiert wird. Hierbei werden zum Beispiel UV-LEDs verwendet. Der Reaktorraum 3 ist mittels Rohren 34, 35 gebildet, die zum Beispiel aus Quarzglas sind. Mittels des Rohres 33 ist die Bestrahlungseinrichtung 2 vom Reaktorraum 3, in welchem das Fluid strömt, getrennt.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Vorrichtung zum Desinfizieren eines Fluids mittels UV-Licht, mit:
- einem Behälter (1);
- einem Reaktorraum (3), welcher in dem Behälter (1) angeordnet und eingerichtet ist, ein zu desinfizierendes Fluid aufzunehmen;
- einem Einlass (4), über welchen das Fluid in dem Reaktorraum (3) eingebracht werden kann;
- einem Auslass (5), über welchen das Fluid den Reaktorraum (3) verlassen kann; und
- einer Bestrahlungseinrichtung (2), die eingerichtet ist, UV-Lichtstrahlen bereitzustellen und zum Desinfizieren des Fluids im Reaktorraum (3) in diesen einzustrahlen;
wobei der Reaktorraum (3) mit dem Einlass (4) und dem Auslass (5) eingerichtet ist, das Fluid mittels einer turbulenten Strömung von Einlass (4) zum Auslass (5) zu transportieren und eine das Ausbilden der turbulenten Strömung unterstützende Fluidleiteinrichtung (9) vorgesehen ist, welche in einem Reaktorraumbereich benachbart zum Einlass (4) ein Fluidleitelement (10) aufweist, das eine nicht turbulente Strömung zum Auslass (5) hin minimierend und hierdurch die turbulente Strömung verstärkend ausgebildet ist.

2. Vorrichtung nach Anspruch 1, **dadurch gekennzeichnet, dass** das Fluidleitelement (10) in Bezug auf den Auslass (5) von diesem beabstandet in einem distalen Bereich des Rohrabschnitts (6) angeordnet ist.

3. Vorrichtung nach Anspruch 1 oder 2, **dadurch gekennzeichnet, dass** das Fluidleitelement (10) in den Reaktorraum (3) hineinragende und dort freistehend angeordnete Blattelemente (11) aufweist.

4. Vorrichtung nach mindestens einem der vorangehenden Ansprüche, **dadurch gekennzeichnet, dass** das Fluidleitelement (10) in dem Reaktorraum (3) dem Einlass (4) gegenüberliegend angeordnet ist.

5. Vorrichtung nach mindestens einem der vorangehenden Ansprüche, **dadurch gekennzeichnet, dass** das Fluidleitelement (10) in dem Reaktorraum (3) verlagerbar angeordnet ist.

6. Vorrichtung nach Anspruch 5, **dadurch gekennzeichnet, dass** das Fluidleitelement (10) in dem Reaktorraum (3) im eine Drehachse drehbar angeordnet ist.

7. Vorrichtung nach mindestens einem der vorangehenden Ansprüche, **dadurch gekennzeichnet, dass** der Auslass (5) an einem Rohrabschnitt (6) angeordnet ist.

8. Vorrichtung nach Anspruch 7, **dadurch gekennzeichnet, dass** der Auslass (5) von einem Wandabschnitt des Reaktorraums (3) ausgehend in den Reaktorraum (3) hineinragend gebildet ist und der Auslass (5) an einem in den Reaktorraum (3) hineinragenden Ende des Rohrabschnitts (6) angeordnet ist.

9. Vorrichtung nach Anspruch 7 oder 8, **dadurch gekennzeichnet, dass** das Fluidleitelement (10) um den Rohrabschnitt (5) herum ausgebildet ist.

10. Vorrichtung nach mindestens einem der Ansprüche 7 bis 9, **dadurch gekennzeichnet, dass** der Auslass (5) an dem Rohrabschnitt (6) im Bereich einer Stirnfläche angeordnet ist.

11. Vorrichtung nach mindestens einem der Ansprüche 7 bis 10, **dadurch gekennzeichnet, dass** das Fluidleitelement (10) eine nicht turbulente Strömung entlang einer äußeren Oberfläche des Rohrabschnitts (6) zum Auslass (5) hin minimierend und hierdurch die turbulente Strömung verstärkend ausgebildet ist.

12. Verfahren zum Desinfizieren eines Fluids mittels UV-Licht, mit:
- Bereitstellen einer Vorrichtung zum Desinfizieren eines Fluids mittels UV-Licht nach mindestens einem der vorangehenden Ansprüche;
- Einbringen des Fluids durch einen Einlass (4) in einem Reaktorraum (3) eines Behälters (1) der Vorrichtung;
- Transportieren des Fluids in dem Reaktorraum (3) zu einem Auslass (5); und
- Desinfizieren des Fluids im Reaktorraum (3) mittels UV-Lichtstrahlen, die von einer Bestrahlungseinrichtung (2) in den Reaktorraum (3) eingestrahlt werden;
wobei das Fluid in dem Reaktorraum mittels einer turbulenten Strömung vom Einlass (4) zum Auslass (5) transportiert wird und hierbei eine Fluidleiteinrichtung (9) das Ausbilden der turbulenten Strömung unterstützt, bei der mittels eines Fluidleitelements (10) in einem Reaktorraumbereich benachbart zum Einlass (4) eine nicht turbulente Strömung zum Auslass (5) hin minimiert und hierdurch das Ausbilden der turbulenten Strömung verstärkt wird.
